# EUROPEAN PATENT APPLICATION

(11) **EP 2 130 833 A1**
(43) Date of publication of application: **09.12.2009**
(21) Application number: 08010275.9
(22) Date of filing: 05.06.2008
(51) Int. Cl.: C07F 9/54, C07C 1/34

(54) **Process for the preparation of zeacarotenes**

(71) Applicant: DSM IP Assets B.V., 6411 TE Heerlen (NL)
(72) Inventor: Simon, Werner, 4125 Riehen (CH); Wegert, Anita, 52066 Aachen (DE); Puhl, Andreas, 79730 Murg (DE)

(57) **Abstract**

The present invention relates to process for the preparation of zeacarotenes and to intermediates in the process for the preparation of zeacarotenes.

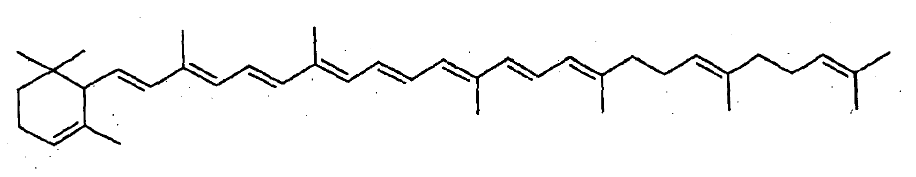

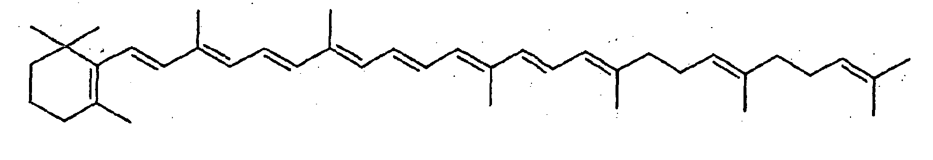

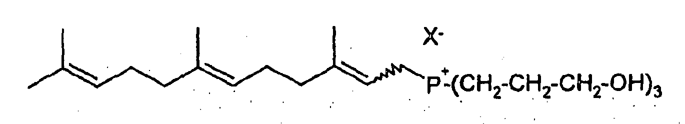

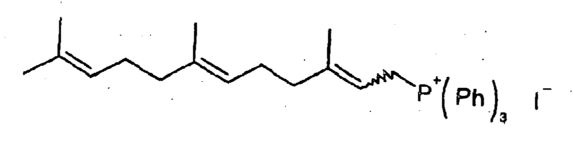

## Description

The present invention relates to a process for the preparation of zeacarotenes and to intermediates in the process for the preparation of zeacarotenes.

Zeacarotenes belong to the family of carotenes, a large group of naturally occurring hydrocarbons.

Zeacarotenes, in particular α-zeacarotene and β-zeacarotene are useful as coloring agents for food or pharmaceutical compositions, as e.g. described in WO 03/022071. They can be used to impart a yellow to greenish-yellow colour to food products such as beverages, sweet and dairy products and pharmaceutical compositions.

α-Zeacarotene, which is also known as 7',8'-dihydro-δ-carotene, is a compound of the formula (1).

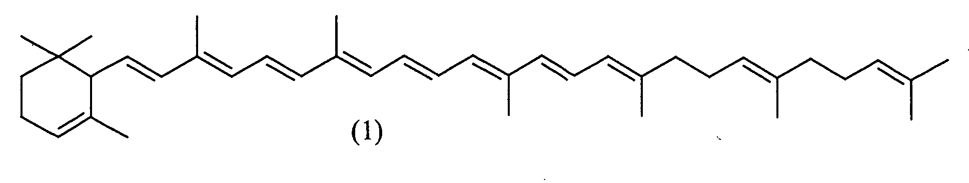

β-Zeacarotene, which is also known as 7',8'-dihydro-γ-carotene, is a compound of the formula (2).. 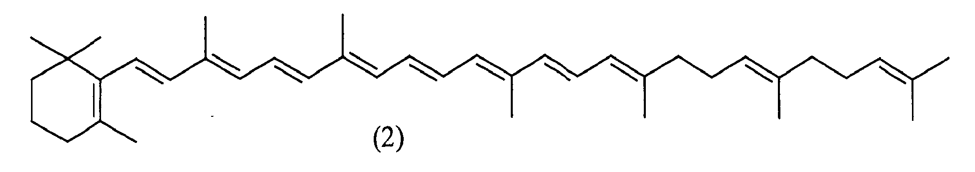 These compounds are structurally very closely related in that the sole difference is the position of the double bond in the cyclohexene ring component.

For most members of this group of carotenes chemical or biochemical processes for the production are known. However, until now, no acceptable chemical synthesis of zeacarotenes is known, by which zeacarotenes can be obtained in acceptable yield and purity.

In the normally applied synthesis of carotenes and carotenoid derivatives the so-called Wittig reaction is frequently applied.

For example WO 2006/039685 discloses methods used for synthesizing intermediates for use in the synthesis of carotenoids and carotenoid analogues, and/or carotenoid derivatives. Wittig reactions are applied to build up the carbon backbone of the carotenoids.

WO 2005/058924 discloses methods for producing phosphonium salts which are intended as starting materials or intermediates, respectively, in the synthesis of unsaturated organic compounds by Wittig reaction. The poor solubility of the phosphonium salts in organic solvents is mentioned as problem. This problem is said to be solved by a special ternary mixture of solvents. The phosphonium compounds used are generally trialkyl-, trialkenyl- or triarylphosphines, preferably triphenylphosphine.

WO 2005/058811 discloses a method for producing vitamin A-acetate. The phosphine compound used is triphenylphosphine.

WO 2005/042446 discloses a method for the production of phytofluene. A Wittig reaction is applied to build up the carbon backbone of the phytofluene, wherein triarylphosphine compounds are used.

DE 100 09 459 discloses a method for the preparation of phosphonium salts. In particular vinyl-ψ-ionol (3,7,11-Trimethyldodeca-1,4,6,10-tetraen-3-ol) is reacted with a triarylphosphine to obtain the corresponding phosphonium salts.

EP 1 092 709 discloses a process for producing β-carotene. The process is characterized by reacting a sulfonated aldehyde with a phosphonium salt, which is also sulfonated to build up the carbon backbone of the β-carotene. However, the use of sulfone derivatives in a process for the preparation of carotenes is not advantageous as the synthesis of these intermediates is complicated by the additional reaction steps necessary to introduce and to remove the sulfone groups. Further additional salt formation is disadvantageous.

EP 1 072 589 discloses a process for producing lycopene and intermediates thereof, wherein also sulfonated intermediates are used as starting materials in the Wittig reaction.

Accordingly there is still a need for a synthesis of carbon backbones of carotenes, in particular zeacarotenes, such as α-zeacarotene or β-zeacarotene, which avoids prior art problems. In particular, there is a need for a process for the preparation of zeacarotenes, which requires less reaction steps and provides higher yields.

It has now been found that several of the prior art problems can surprisingly be overcome by the preparation of a modified Wittig salt for application in the Wittig reaction to form the carbon backbone of carotene derivatives.

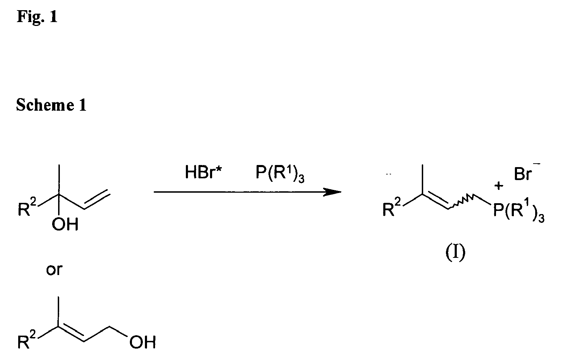

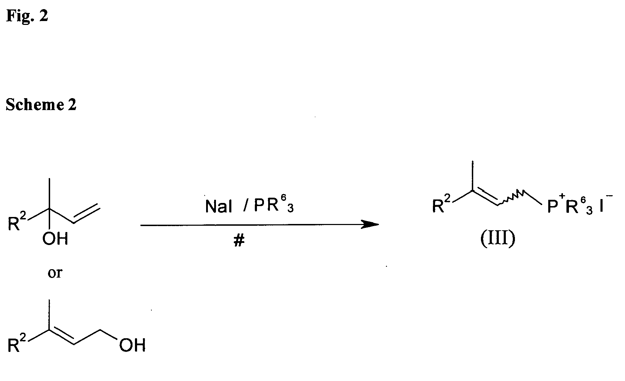

In particular, it has been found that Wittig salts of formulae (I) and (III) to be used in the Wittig reaction to form the carbon backbone of a carotene derivative can advantageously be synthesized from allylic alcohols preferably according to the reaction schemes 1 or 2 depicted in Fig. 1 and Fig 2, respectively.

* The HBr is preferably used in (glacial) acetic acid. Furthermore a solvent (e.g. CH₂Cl₂) and a water capturing agent such as e.g. trimethyl-ortho-formate, methoxy propene, acetic acid anhydride or dimethoxy propane are present.

# An acid is preferably also present. More preferably the acid is HCO₂H or H₃CCO₂H.

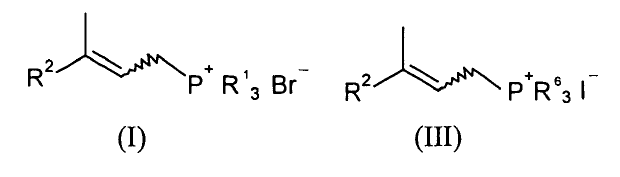

In the above formulae, R¹ and R⁶ independently are C₂₋₆-alkyl, which may be substituted, or aryl, which may be substituted, R² is alkyl, cycloalkyl, alkenyl or cycloalkenyl. Preferred meanings of R¹, R² and R⁶ are defined below.

Within the present application the notation R₃, e.g. in R¹₃ and R⁶₃, also comprises the embodiment wherein the three residues R, e.g. R¹ and R⁶, respectively, are different within one molecule, e.g. such that R₃ describes three different alkyl residues or two optionally different alkyl residues and one aryl residue, or one alkyl residue and two optionally different aryl residues, or three different aryl residues. In other words, R₃ denotes R' R" R''', wherein R', R" and R''' are independently selected from the residues R can be selected from.

The advantage of the reaction according to scheme 1 is that it provides high yields and excellent E/Z ratios of the obtained Wittig salts and it further can be optimized either to higher yields of the Wittig salts or to more excellent E/Z ratios of the Wittig salts obtained.

The advantage of the reaction according to scheme 2 is that the Wittig salt (i.e. iodide) can be obtained in excellent yields and can easily be crystallized, which has the further advantages of high purity and defined E/Z ratios.

Therefore, the present invention relates to a process for the preparation of a compound of formula (I) 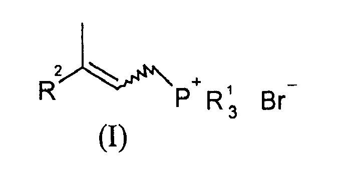 which process comprises the step of reacting a compound of the formulas II or IV 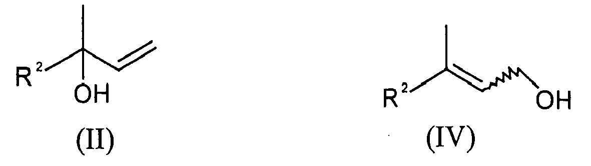 with HBr and PR¹₃,
wherein R¹ is an optionally substituted C₂₋₆ alkyl or an optionally substituted aryl and R² is alkyl, cycloalkyl, alkenyl, or cycloalkenyl.

The compound of formula (I) prepared by the process of the present invention is a Wittig salt which may be applied in the Wittig reaction, particularly to build up a carbon backbone in the synthesis of carotene derivatives.

The HBr used in the process of the preparation of a compound of formula (I) of the present invention can be used in any suitable form to allow the conversion of an allylic alcohol into the corresponding bromide. Preferably, the HBr used is in form of a HBr/glacial acetic acid solution (e.g. 33 weight-%) or most preferred as gaseous HBr.

R¹ is an optionally substituted alkyl group having 2-6 carbon atoms, such as ethyl, n-propyl, isopropyl, n-butyl, isobutyl, tert-butyl, n-pentyl or n-hexyl, or R¹ is an aryl group, such as phenyl or naphthyl, which aryl may be substituted by at least one alkyl group (e.g. C₁-C₃-alkyl). Preferably, R¹ is n-propyl, n-butyl or a phenyl group. The group R¹ can optionally be substituted, preferably substituted by hydroxy, halogen, or a sulfonyl group, and can in particular be 3-hydroxypropyl. Most preferably R¹ is n-butyl or phenyl, optionally substituted with a C₁-C₃-alkyl (e.g. a methyl, ethyl, n-propyl, isopropyl) or with a C₁₋₃-alkoxy group (e.g. methoxy, ethoxy, n-propoxy, or isopropoxy group).

R² is generally a branched or non-branched alkyl, branched or non-branched cycloalkyl, branched or non-branched alkenyl, or branched or non-branched cycloalkenyl, such as straight C₁-C₃₀-, branched C₂-C₃₀- or cyclic C₃-C₃₀-alkyl, preferably C₂-C₁₆-alkyl, or such as straight C₂-C₃₀-, branched C₃₋C₃₀ or cyclic C₅-C₃₀-alkenyl, preferably C₃-C₁₆-alkenyl, respectively.

It is more preferred that R² is a group of formula (XI) 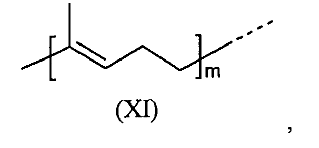 wherein m is an integer from 1-3, in particular m = 2. The compound of formula (II) is preferably E-Nerolidol, and the compound of formula (IV) is preferably E,E- or E,Z-Farnesol, and the corresponding compound of formula (I) is a E,E- or E,Z-Farnesylphosphonium salt, respectively. The compound E-Nerolidol is most preferably used in the process for the preparation of a compound of formula (I) of the present invention.

The reaction for the preparation of a compound of formula (I) of the present invention is generally conducted in a suitable solvent for producing Wittig salts, e.g. CH₃CN, toluene, CH₂Cl₂, diethyl ether, ethyl acetate, or mixtures thereof, preferably in CH₂Cl₂. The starting materials are typically solved in these solvents, preferably under an inert gas atmosphere (e.g. N₂, Ar). It has been found that the presence of water significantly reduces the yield. Therefore preferably, a water capturing agent is added before addition of the HBr as a HBr / glacial acetic acid mixture or preferably as gaseous HBr. Examples of water capturing agents are trimethyl-ortho-formate, methoxy propene, acetic acid anhydride or dimethoxy propane. The HBr is preferably added at a temperature in the range of from 0 to 30°C. After completion of the reaction, which may be followed by suitable methods, (e.g. chromatographic measurements), the phosphine compound, i.e. the PR¹₃ compound, is usually added. The resulting Wittig salts can be extracted by methods known in the art, e.g. by extraction of the aqueous phases with an organic solvent, e.g. dichloromethane (CH₂Cl₂), or by crystallization.

The process according to the present invention for the preparation of a compound of formula I may be part of a process for the preparation of carotene derivatives, in particular for the preparation of α-zeacarotene or β-zeacarotene.

The present invention also relates to a process for the preparation of a compound of formula (III) 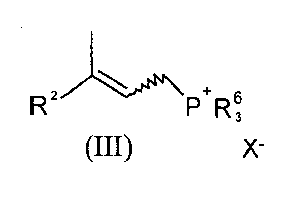 wherein R² is C₁₋₃₀-alkyl, cycloalkyl, alkenyl, or cycloalkenyl, R⁶ is an optionally substituted C₂₋₆ alkyl or an optionally substituted aryl, and X⁻ is the counterion,
which process comprises the step of reacting a compound of the formulas (II) or (IV) 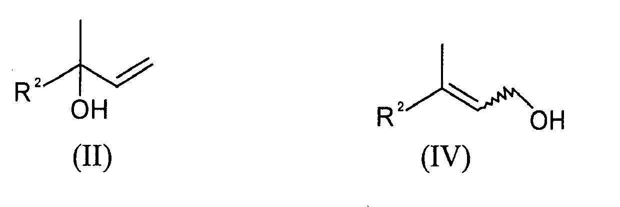 with MX and PR⁶₃, wherein M⁺ is a cation.

M⁺ is a suitable cation for the preparation of the compound of formula (III), such as H⁺, Na⁺, K⁺, preferably Na⁺.

X⁻ is a counterion suitable within a Wittig salt, such as a compound of the formula (III), and which renders the Wittig salt suitable to be used in a Wittig reaction. Such anions are known to a person skilled in the art. Preferably, X⁻ is chloride, iodide, tosylate (⁻OTs, salt of toluene sulfonic acid), mesylate (⁻OMs, salt of methyl sulfonic acid), or triflate (⁻OTf, salt of trifluoromethane sulfonic acid). X⁻ is most preferably iodide (I⁻). X⁻ is preferably not bromide.

R⁶ is an optionally substituted alkyl group having 2-6 carbon atoms, such as ethyl, n-propyl, isopropyl, n-butyl, tert-butyl, n-pentyl or n-hexyl, or R⁶ is an aryl group, such as phenyl or naphthyl, which aryl may be substituted by at least one alkyl group. R⁶ is preferably n-butyl or phenyl, which may be substituted with an C₁-C₃-alkyl (e.g. a methyl, ethyl, n-propyl, isopropyl group) or a C₁-C₃-alkoxy group (e.g. a methoxy, ethoxy, n-propoxy, or isopropoxy group). Most preferably R⁶ is a phenyl group.

The reaction for the preparation of a compound of formula (III) of the present invention is generally conducted in a suitable solvent for producing Wittig salts, e.g. alcohols, esters and amides, preferably CH₃CN, toluene, CH₂Cl₂, diethyl ether, ethyl acetate, or mixtures thereof, more preferably in CH₂Cl₂, ethyl acetate or CH₃CN, in particular CH₂Cl₂. The starting materials are typically solved in those solvents, preferably under inert gas atmosphere. A water capturing agent as described above can be added. After addition of concentrated acid, such as formic acid (∼100%), sulfonic acid (∼95%) or phosphoric acid (80-100%), or of an acid in a concentration and an amount sufficient to prepare HI (hydrogen iodide) from R³I, if L⁻ is I⁻ and if R³ is not H⁺, the phosphine compound, i.e. the PR⁶₃ compound, is added, preferably at a temperature in the range of from 0 to 30°C. After completion of the reaction, which can be followed by suitable methods such as chromatographic measurements, the resulting Wittig salts can be extracted from the reaction solution by methods known in the art, e.g. by extraction of the aqueous reaction solution with an organic solvent, e.g. dichloromethane (CH₂Cl₂). The product can also be crystallized from the crude reaction product, preferably after transfer of the product into a suitable crystallization solvent.

The compounds of formula (I) and (III) are useful as intermediates in the preparation of carotenes, in particular zeacarotenes, such as α-zeacarotene or β-zeacarotene.

Therefore the present invention also relates to a process for the preparation of a compound of formula (V) 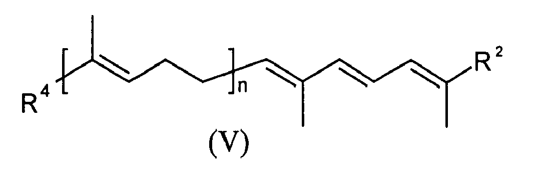 wherein R² is alkyl, cycloalkyl, alkenyl, or cycloalkenyl,
R⁴ is a branched or non-branched alkyl, branched or non-branched cycloalkyl, branched or non-branched alkenyl, or branched or non-branched cycloalkenyl,
and n is an integer from 0 to 3,
which process comprises

### a1) reacting a compound of formulas (II) or (IV)

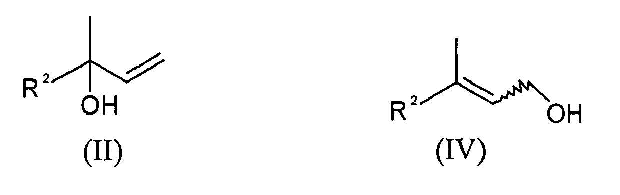

with HBr and PR¹₃,
wherein R¹ is an optionally substituted C₂₋₆ alkyl or an optionally substituted aryl, to give a compound of formula I 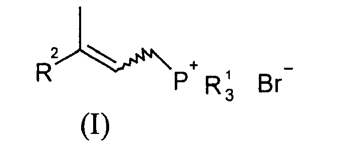 or

### a2) reacting a compound of formulas (II) or (IV)

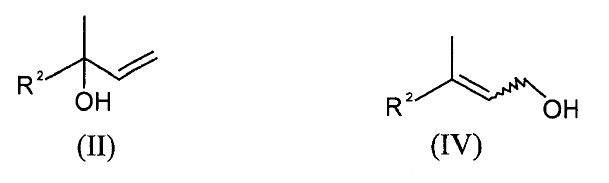

with MX and PR⁶₃,
wherein M⁺ is a cation, R⁶ is an optionally substituted C₂₋₆ alkyl or an optionally substituted aryl and X⁻ is an anion as defined above, to give a compound of formula (III) 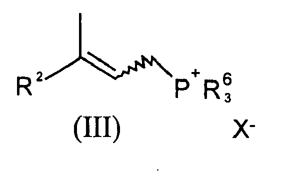 and

### b) reacting the compound of formula (I) or (III) obtained in step a1) or a2) with a compound of formula (VI)

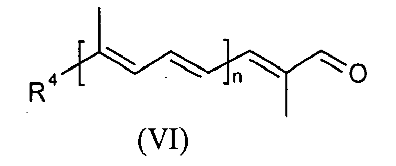

to obtain a compound of formula (V).
R⁴ is generally a branched or non-branched alkyl, branched or non-branched cycloalkyl, branched or non-branched alkenyl, or branched or non-branched cycloalkenyl. R⁴ may be C₁-C₁₆ alkyl, preferably C₁-C₁₂ alkyl. R⁴ may be linear C₂-C₁₆ alkenyl or branched C₃-C₁₅ alkenyl, preferably C₃-C₁₂ alkenyl. R⁴ may further be C₃-C₁₆ cycloalkyl or cycloalkenyl, preferably C₄-C₁₂ cycloalkyl or cycloalkenyl, most preferably C₆-C₁₂ cycloalkyl or cycloalkenyl. Most preferably, R⁴ is

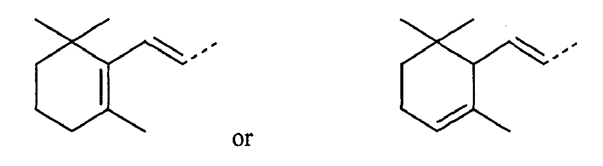

In the compounds of formula (V) and (VI) n is preferably an integer from 1 to 3, more preferably n=1 or n=2, most preferably n=2.

R² and R⁴ in the compound of formula (V) are defined as above, preferably R² and R⁴ of the compound of formula (V) are such that the compound of formula (V) is a carotene derivative, thus R² is preferably a group of formula (XI), 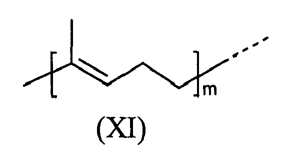 wherein m is an integer from 1 to 3, in particular m = 2, and R⁴ is preferably 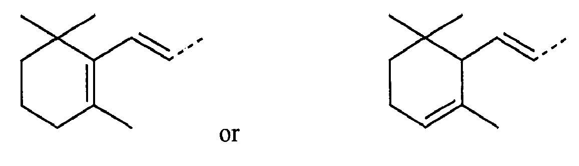 and n = 2.

The compounds of formula (VI) to be used in the process for the preparation of the compound of formula (V) of the present invention can be prepared according to chemical syntheses known in the art, such as disclosed in DE 2 357 753 or O. Isler, et al., Helvetica Chemica Acta, 1959, 42, 847-853.

The compounds of formula (I) and (III) are useful as intermediates for the preparation of the compound of formula (V), and are preferably used in the process for the preparation of the compound of formula (V) of the present invention.

In the process for the preparation of a compound of formula (V) the compounds of formula I or (III) are used, which are obtained by the processes for the preparation of a compound of the formula I or a compound of the formula (III) of the present invention as described above.

In one preferred embodiment of the process for the preparation of a compound of formula (V), the compounds of formulas (I) or (III) obtained in the step a1) or a2) are reacted in step b) without isolation of the compounds of formulas (I) or (III). Thus, the compounds of formula (I) or (III) are obtained by the processes for the preparation of a compound of the formula (I) or (III) as described above and are directly reacted with a compound of formula (VI) without conducting a purification step of the compounds of formula (I) or (III). This embodiment of the process for the preparation of a compound of formula (V) of the present invention is particularly advantageous, as a significant reduction in the preparation steps to obtain a compound of the formula (V) can be achieved.

Preferably the compound of formula (I) or (III) obtained in step a1) or a2) is a compound of formula (I), wherein R¹ is n-butyl or phenyl and R² is defined as above, or a compound of formula (III), wherein R² is defined as above, R⁶ is phenyl and X⁻ is I⁻.

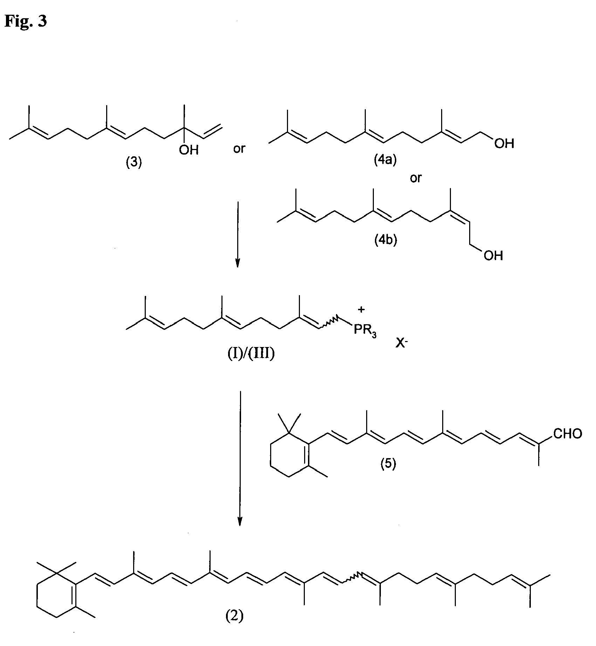

In the most preferred embodiment of the present invention in the process for the preparation of a compound of formula (V), the compound of formula (II) is E-Nerolidol (compound of formula (3)) or the compound of formula (IV) is E-E- or E-Z-Farnesol (compounds of formulae (4a) and (4b), respectively), the compound of formula (VI) is β-Apo-12'-carotenal (compound of formula (5)) or the corresponding isomer (compound of formula (6)), and the compound of formula (V) is α-zeacarotene (compound of formula (1)) or β-zeacarotene (compound of formula (2)) (see Fig. 3). 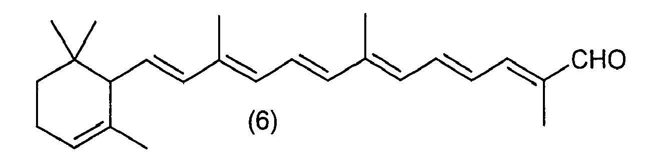 Thus, α-zeacarotene may be prepared in a manner analogous to the preparation of β-zeacarotene as shown in Fig. 3 by using the compound of formula (6) instead of the compound of formula (5).

The reaction for the preparation of the compound of formula (V) of the present invention may be carried out in usual solvents for carrying out Wittig reactions, such as alcohols, esters, amides, preferably methanol, ethanol, CH₂Cl₂, hexane, cyclohexane, THF (tetrahydrofurane), acetic ester, CH₃CN, ethanol or DMF (dimethylformamide), or mixtures thereof, more preferably in CH₂Cl₂ or hexane, in particular in hexane. The suspension resulting from applying the starting materials into the solvent can optionally be stirred, preferably under inert atmosphere. The alkaline compound, which is usually applied to conduct the Wittig reaction, is added. As alkaline compound, all usually applicable alkaline substances for conducting the Wittig reaction can be applied, preferably a methanolate or ethanolate, in particular sodium methanolate. After addition of the corresponding aldehyde and completion of reaction, solid particles, which may be present, can be filtered off. The reaction product, i.e. the compound of formula (V), can be removed and purified as known in the art. Preferably the reaction product is crystallized from the raw product or from the purified reaction product after some purification step, in particular directly from the raw product. This can e.g. be achieved by addition of further different solvents, in particular those, wherein the product is less soluble.

It has surprisingly been found that by the process for the preparation of the compound of the formula (I), in particular the special choice of a specific bromination reagent instead of the usually applied tribromine-phosphine (Br₃P) for the bromination of the allylic alcohol of formulas (II) or (IV), the compound of formula (I) can be obtained in excellent yield. Additionally said compound of formula (I) is advantageous when used in a Wittig reaction as a high E/Z ratio of the double bond formed in said Wittig reaction can be obtained. Preferably a E/Z ratio of the double bond formed of from 3:1 to 20:1 of the corresponding product, most preferred of from 3:1 to 5:1, can be obtained.

Further it has surprisingly been found, that the compound of the formula (III), which is produced by the process for the preparation of the compound of formula (III) according to the present invention, can easily be obtained in pure form by crystallization of the product from the raw reaction product. In particular the resulting Wittig salt, preferably the phosphonium iodide, can be easily crystallized from the raw product thereby obtaining the pure compound of formula (III). Additionally said compound is advantageous when used in a Wittig reaction as a high E/Z ratio of the double bond formed in said Wittig reaction can be obtained. Preferably an E/Z ratio of the double bond formed of from 2:1 to 10:1, most preferred of from 3:1 1 to 5:1, of the corresponding product can be obtained.

Therefore, in the process for the preparation of a compound of formula (V), the compounds of formula I and formula (III), which are preferably obtained by the process for the preparation of the compound of formula (I) and formula (III) of the present invention, are preferably used to prepare a compound of the formula (V), wherein a high E/Z ratio of the resulting carbon double bond created in the Wittig reaction can be achieved.

Thus by the processes of the present invention intermediates and processes are provided, which can advantageously be applied to obtain a carotene derivative, in particular a Zeacarotene derivative, which has an advantageously high E/Z ratio of the double bond formed in the Wittig reaction. Thereby, e.g. an E/Z ratio of 20:1 can be achieved in zeacarotenes (referring to the double bond at the 11'-12' position) using trialkylphosphines, while an E/Z ratio of 5:1 to 7:1 can be achieved when using triphenylphosphine.

The process for the preparation of a compound of formula (V) of the present invention is also advantageous, as it is not necessary to apply the Wittig salts in a purified form, but can be prepared, (preferably according to a process to prepare a Wittig salt of the present invention), and can directly be reacted with a carbonyl compound, (preferably a compound of formula (VI)), without isolation and / or purification of the Wittig salt. Thus the complete reaction sequence starting from compounds of formula (II) or (IV) to obtain a compound of formula (V) can be conducted within a few hours instead of days, which are necessary when a Wittig-reaction is conducted where the Wittig salt has to be isolated and/or purified in between. This allows the production of zeacarotene from nerolidol or farnesol in a "one-pot reaction."

However, in order to further increase the yield, the Wittig salts are preferably purified before reaction with the carboxyl compound.

Further the process for the preparation of a compound of formula (V) of the present invention has the advantage that the aldehyde used, which is preferably β-Apo-12'-carotenal (C₂₅-aldehyde), can be applied in a purity of about 90% (sum of E + Z isomers) which is sufficient that the compound of formula (V) can be obtained in crystal form in excellent yield.

The process for the preparation of a compound of formula I of the present invention further has the advantage that the compound PBr₃, which is usually applied for bromination of allylic alcohols for the preparation of Wittig salts and is disadvantageous, can be avoided by the process for the preparation of a compound of formula I of the present invention.

The present invention further relates to the compounds of formula (VIII) 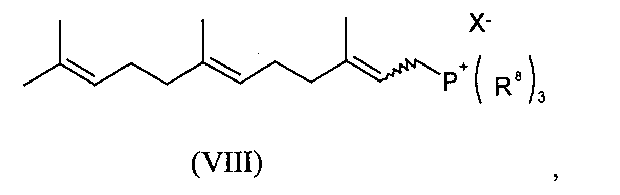 wherein X⁻ is a counterion as defined above including bromide, preferably X⁻ is chloride, bromide or iodide, and R⁸ is optionally substituted alkyl,
to the compounds of the formula (IX) 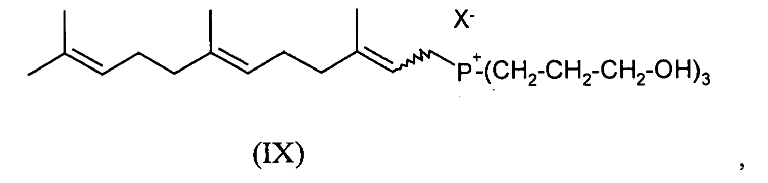 wherein X⁻ is a counterion as defined above,
and to the compounds of formula (X) 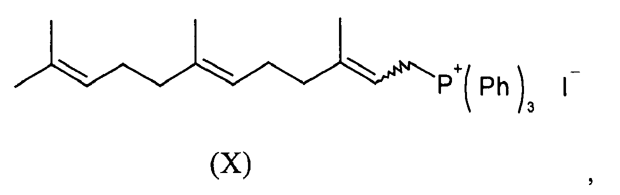 wherein "Ph" is a phenyl group.

These compounds are useful intermediates in the preparation of zeacarotenes, in particular α-zeacarotene or β-zeacarotene.

R⁸ is an optionally substitued alkyl group having 2-6 carbon atoms, such as ethyl, n-propyl, isopropyl, n-butyl, tert-butyl, n-pentyl or n-hexyl, preferably n-butyl. Possible substituents of the alkyl group are at least one hydroxy group, halogen or sulfonyl group.

Within this application the wavy line depicted by 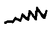 indicates a single bond and the stereochemistry relating to the double bond bound therewith is E or Z or a mixture thereof.

The present invention will now be further illustrated by the following examples which are not intended to be limiting.

### Examples

For all examples described below E-Nerolidol (>98% E, designated "Nerolidol") and E-E-farnesol (>94% E,E) (designated "Farnesol") was used.

### Example 1: Farnesyltriphenylphosphoniumiodide (compound of formula (III))

10.0 g of Nerolidol were dissolved in 50 ml of CH₂Cl₂ at 25°C. Further, 5.4 g of acetic acid, 11.79 g of TPP (triphenylphosphine) and 7.41 g NaI were added. The mixture was reacted for 24 hours at 25°C under continuous addition of 9.84 ml of trimethyl-o-formate and 1.7 ml of formic acid. After extraction with saturated NaHCO₃-solution and evaporation of the solvent, 20.07 g of a crystalline product were obtained (yield: 75%). The crystals had a purity of ∼100% E+Z. The E/Z-ratio was 2.17 : 1.

### Example 2: Farnesyltris(hydroxyprolpyl)phosphoniumbromide (compound of formula (I))

5 g of Nerolidol (22.48 mmol) were dissolved in 50 ml CH₂Cl₂ and stirred for 15 minutes under argon. After addition of 3.7 ml of trimethyl-o-formate (1.05 eq.), 5.7 g HBr / glacial acetic acid (33.5%) were fed at 25°C dropwise to the solution. After 1 hour at 25°C, the reaction mixture was extracted with 50 ml of saturated NaHCO₃ solution (backextraction with CH₂Cl₂ and saturated NaCl solution). The combined organic phases were dried over Na₂SO₄. To this solution 5.2 g of tris(hydroxypropyl)phosphine in 20 ml of ethanol were added at 25°C. After 30 minutes the solution was evaporated to dryness. The weight-yield of the crude product was ∼ 100%. The ratio of the E/Z-phosphoniumsalt isomers was 9.4 : 1 according to HPLC/MS.

### Example 3: Famesyltris(n-butyl)phosphoniumbromide (compound of formula (I))

44.97 mmol of Farnesol (10 g) were dissolved in 50 ml CH₂Cl₂ and stirred for 15 minutes under argon. 7.38 ml of trimethyl-o-formate (1.5 eq.) and 8.20 ml HBr / glacial acetic acid (33.5%, 1.05 eq.) were added at 0 - 10°C. After stirring for 1 hour at 5°C, 1.1 eq. (10 g) of tri-n-butylphosphine (TBP) were added and stirring was continued for another hour at 5°C. The organic phase was extracted subsequently with saturated NaHCO₃ and NaCl solution (back extraction with dichloromethane). The combined organic phases were dried over Na₂SO₄ and the solvent was evaporated. The weight-yield of crude product was 98.6%, the E/Z-ratio was 19.6 : 1.

### Example 4: β-zeacarotene (compound of formula (V))

6.91 mmol (3.37 g) of Farnesyltri(n-butyl)phosphoniumbromide and 0.8 eq. of β-apo-12'-carotenal ("C25-aldehyde", 2.04 g, purity: 95.23%) were suspended in 30 ml of cyclohexane. 1.5 eq. of NaOMe (1.89 ml, 30% in MeOH) were added dropwise within 1 hour at 5°C. After another time of 6 hours at 25°C, the reaction was completed according to TLC (thin layer chromatography). The reaction mixture was then extracted with saturated NaCl solution, the resulting organic phase was dried over Na₂SO₄ and then evaporated to dryness (chemical yield 78%). The residue was dissolved in 30 g 2-butanol / n-hexane (5 : 1) and then 24 g of methanol were added at 25°C within 30 minutes. The resulting crystal suspension was cooled down to 0°C, kept at this temperature for 2 hours, filtered and the resulting crystals dried at 30°C / 10 mbar. The yield of crystalline all-E-β-Zeacarotene was 20%.

### Example 5: β-zeacarotene, one pot compound of formula (V))

22.48 mmol (5 g) of Nerolidol were dissolved in 55 ml THF (tetrahydrofuran) and stirred for 15 minutes under argon. 3.7 ml of trimethyl-o-formate and 5.86 ml HBr / glacial acetic acid (33.5%) were added in 2 minutes at 10°C. After stirring for 1 hour, 4.55 g tri-n-butylphosphine and 5.76 g of C25-aldehyde (β-apo-12'-carotenal, purity: 95.8%) in 5 ml of THF were added. After that, 7.31 g solid sodium methanolate were added and the temperature was allowed to rise from 10°C up to 55°C for a short time. The mixture was then stirred for 1.5 hours at 25°C. After filtration, the solution is evaporated to a residue of 20 g. Crystallization of this residue from a THF / Methanol / Ethanol-mixture (1 : 2 : 2) gave 5.46 g of E/Z-β-Zeacarotene (yield 57%). The E/Z-ratio was 2.15 : 1).

### Example 6: Farnesyltriphenylphosphoniumbromide (compound of formula III)

157.4 g Triphenylphosphine (TPP, 0.6 mol) and 136.3 g Nerolidol (0.6 mol) were dissolved in 627 ml CH₂Cl₂ at 23°C under argon. The solution was cooled to 17°C and 117.6 ml trimethyl-o-formate (1.075 mol) were added, followed by dropwise addition of 104.3 ml HBr in glacial acetic acid (33.5%) within 15 minutes, whereby temperature rose to 19°C. The solution was stirred at 17°C for 2 hours. 324 ml of aqueous NaOH (20%) were added, whereby temperature rose to 25°C. The solution was further stirred for 30 minutes. The organic phase was extracted 3 times with 300 ml water. The aqueous phases were back-extracted with 200 ml CH₂Cl₂. The combined organic phases were dried over Na₂SO₄ and the solvent was evaporated at 40 °C / max. 180 mbar. The colorless residue had a weight of 396.36 g (chemical yield: 71.9%, E / Z-ratio: 2.8 : 1).
262.64 g of the resulting colourless residue was dissolved in 550 ml isobutanol, whereby 698.92 g of a solution was obtained. Some small amount of solvent was then evaporated in order to remove any residual CH₂Cl₂. Hereby 661.72 g of solution A was obtained, which has been used in the following example 7.

### Example 7: β-zeacarotene (compound of formula (V))

A mixture of 57.5 mmol of Farnesyltriphenylphosphoniumbromide as an isobutanolic solution (132.68g of solution A, containing 23.73% E- + Z-isomers of Farnesyltriphenylphosphoniumbromide) and 6 drops of sodium methanolate (30% in MeOH) were prepared at room temperature under argon, followed by addition of 18.89 g C25-aldehyde (β-apo-12'-carotenal, 92.8%) and 125 ml of isobutanol. Under vigorous stirring the main part of sodium methanolate (total of 13.77 ml NaOMe in MeOH, 30%) was added within 30 minutes while the temperature was kept between 19 and 23°C. The suspension was further stirred at 40°C for 2 hours, followed by cooling down to 23°C and dropwise addition of 134 ml MeOH within 15 minutes. After further stirring for 2 hours the suspension was filtered and the obtained crystals were washed with MeOH (2 x 50 ml) and dried over night in a vacuum dryer at 30°C.

The weight yield was 14.5 g of red crystals (53.74% based on C25-aldehyde). The crystals contained 76.4% all-E-β-Zeacarotene and a total of 15.5% of Z-isomers of β-Zeacarotene.

### Example 8: Farnesyltriphenylphosphoniumbromide (compound of formula (III))

A mixture of 78.69 g triphenylphosphine (TPP, 0.3mol) and 68.15 g Nerolidol (0.3 mol) dissolved in 313 ml CH₂Cl₂ were prepared in a 4-necked round bottomed flask under Argon at 23°C. The solution was cooled down to 17°C, 58.80 ml trimethyl-o-formate (0.53 mol) were added, followed by slowly feeding of 52.13 ml HBr / glacial acetic acid (33%) within 14 minutes. The temperature was allowed to rise up to 21 °C. The solution was stirred for 2 hours at 17°C. Completion of the reaction was controlled by HPLC. Afterwards, 162 ml NaOH (20%) was added within 8 minutes, whereby the temperature rose to 18°C. The solution (pH 7.0) was stirred for 30 minutes. The organic phase was then extracted 3 times with 150 ml water (the aqueous phases were back-washed with 50 ml CH₂Cl₂. The combined organic phases were dried over Na₂SO₄ and the solvent was evaporated at 40°C / max. 250 mbar to a residual weight of 190.09 g. This solution was diluted with 445 ml ethyl acetate. The resulting crystal suspension was stored for 15 hours at 4°C, filtered and the obtained crystals were washed with cold (0°C) ethyl acetate. The crystals were dried over night in a vacuum dryer at 30°C. 107.36 g of white crystals were obtained. The weight yield was 65.4%, the E/Z ratio was 3.5 : 1.

### Example 9: β-zeacarotene (compound of formula (V))

3.54 g of Farnesyltriphenylphosphoniumbromide (6 mmol) was dissolved in 16.2 ml isobutanol under Argon. 2 drops of sodium methanolate (30% in MeOH) were added until the color changes to yellow. Then 1.83 g C25-aldehyde (β-apo-12'-carotenal, 95.72 %) in 10.0 ml of isobutanol were added. The solution was stirred at room temperature and the main part of sodium methanolate (total of 1.1 ml NaOMe in MeOH, 30%) was fed within 1 hour. After the addition is complete, the temperature was increased to 40°C for 1.5 hours under stirring. The suspension was then cooled down to room temperature and 13.0 ml of methanol were fed within 90 minutes. The suspension was cooled to 2°C and stirred for 1.5 hrs before being filtered. The crystals were washed 2 times with 3 ml of cold MeOH (0°C) before being dried over night in a vacuum dryer at 10mbar / 30°C. 1.97 g of red crystals were obtained. The weight-yield was 73.1%, the E / Z-ratio: 3.6 : 1. The total content of E/Z-β-Zeacarotene was 98 weight-%.

### Example 10: β-zeacarotene (compound of formula (V))

3.39 g of Farnesyltriphenylphosphoniumbromide (5.8 mmol) was dissolved in 17 ml ethanol under argon. 3 drops of sodium ethanolate (21.6% in ethanol) were added until the color changes to yellow. Then 1.89 g C25-aldehyde (β-apo-12'-carotenal, 92.79%) in 10.0 ml of ethanol were added. The suspension was stirred at room temperature and the main part of sodium ethanolate (total of 2.2 ml NaOEt in ethanol, 21.6 %) was fed within 30 minutes. After the addition was complete, the temperature was increased to 40°C and stirring continued for 5 hours. The suspension was then cooled down to 25°C. After stirring for 30 minutes at 25°C the suspension was filtered. The crystals were washed 2 times with 3 ml of ethanol, before being dried over night in a vacuum dryer at 10mbar / 30°C. 2.12 g of red crystals were obtained. The weight-yield was 78.7 %, the E / Z-ratio: 2.5 : 1. The total content of E/Z-β-Zeacarotene was 93 weight-%.

## Claims

1. A process for the preparation of a compound of formula (I) 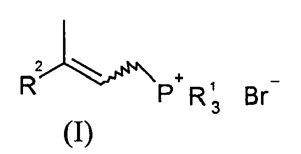 which process comprises the step of reacting a compound of the formula (II) or (IV) 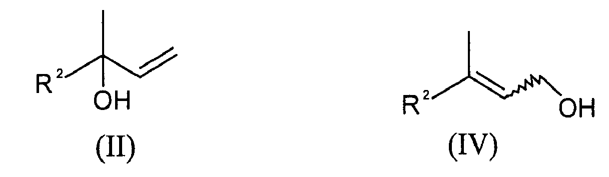 with HBr and PR¹₃,
wherein R¹ is optionally substituted C₂₋₆ alkyl or optionally substituted aryl; and
R² is alkyl, cycloalkyl, alkenyl or cycloalkenyl.

2. The process according to claim 1, wherein R¹ is n-propyl, n-butyl, 3-hydroxypropyl or phenyl and/or R² is a group of formula (XI) 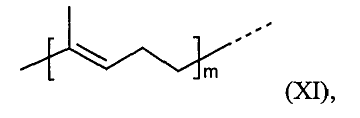 wherein m is an integer from 1-3.

3. A process for the preparation of a compound of formula (III) 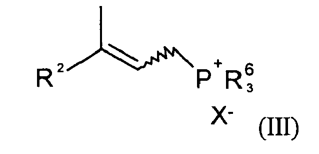 which process comprises the step of reacting a compound of the formula (II) or (IV) 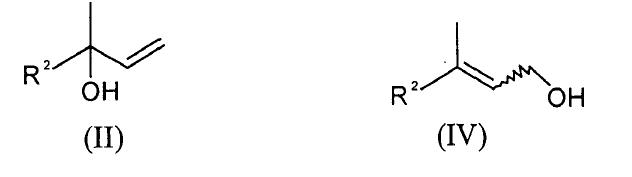 with MX and PR⁶₃,
wherein R² is alkyl, cycloalkyl, alkenyl or cycloalkenyl,
R⁶ is optionally substituted C₂₋₆ alkyl or optionally substituted aryl,
M⁺ is a cation and X⁻ is an anion.

4. The process according to claim 3, wherein R² is a group of formula (XI) 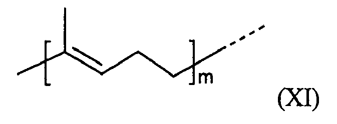 wherein m is an integer from 1-3.

5. A process for the preparation of a compound of formula (V) 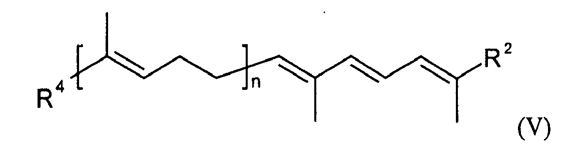 which process comprises
a1) reacting a compound of formula (II) or (IV) 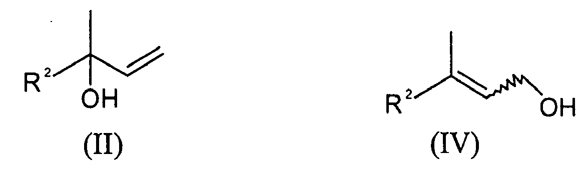 with HBr and PR¹₃,
to give a compound of formula I 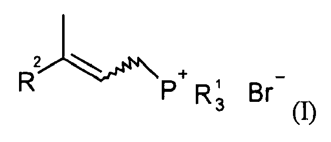 or
a2) reacting a compound of formula (II) or (IV) 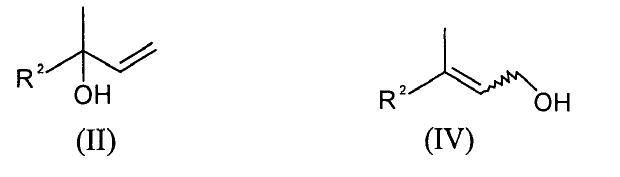 with MX and PR⁶₃,
to give a compound of formula (III) 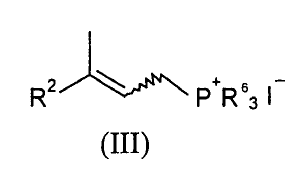 and
b) reacting the compound of formula (I) or (III) obtained in step a1) or a2)
with a compound of the formula (VI) 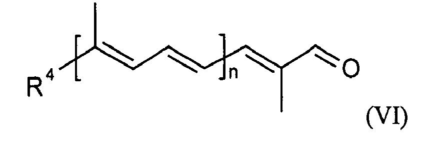 to obtain a compound of the formula (V),
wherein R¹ and R⁶ are independently from each other optionally substituted C₂₋₆ alkyl or optionally substituted aryl,
R² and R⁴ are independently from each other branched or non-branched alkyl, branched or non-branched cycloalkyl, branched or non-branched alkenyl, branched or non-branched cycloalkenyl,
M⁺ is a cation, X⁻ is an anion and n is an integer from 0-3.

6. The process according to claim 5, wherein R⁴ is

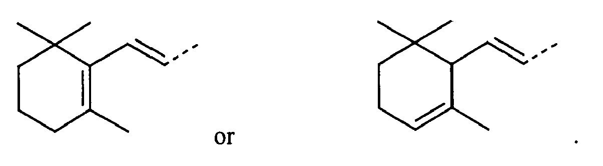

7. The process according to claim 5 or 6, wherein n is 1 or 2.

8. The process according to any one of claims 5-7, wherein the compound of formula (I) or (III) obtained in step a1) or a2) is reacted in step b) without isolation of the compounds of formulas (I) or (III).

9. The process according to any one of claims 5-8 wherein the compound of formula (I) or (III) is a compound of formula I, wherein R¹ is n-butyl or 3-hydroxypropyl, and R² is alkyl, cycloalkyl, alkenyl or cycloalkenyl, or a compound of formula (III), wherein R² is alkyl, cycloalkyl, alkenyl or cycloalkenyl, R⁶ is a phenyl and X⁻ is I⁻.

10. The process according to claim 9, wherein R² is a group of formula (XI) 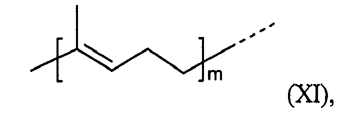 wherein m is an integer from 1-3.

11. The process according to any of claims 5-10, wherein the compound of formula (II) is E-Nerolidol or the compound of formula (IV) is E-E- or E-Z-Farnesol, the compound of formula (VI) is β-Apo-12'-carotenal and the compound of formula (V) is α-zeacarotene or β-zeacarotene.

12. A compound of the formula (VIII) 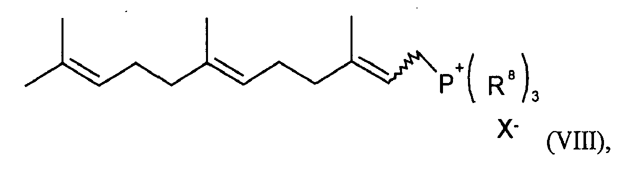 wherein X⁻ is an anion and R⁸ is optionally substituted alkyl.

13. A compound of formula (IX) 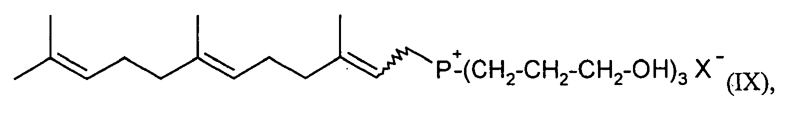 wherein X is Cl, Br or I.

14. A compound of formula (X)

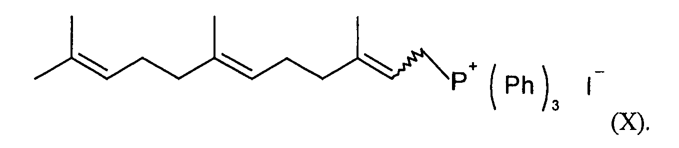

15. The use of a compound of any one of claims 12-14 for the preparation of a zeacarotene.
